# NEW EUROPEAN PATENT SPECIFICATION

(11) **EP 0 522 368 B2**
(45) Date of publication and mention of the opposition decision: **15.03.2000**
(45) Mention of the grant of the patent: 19.03.1997
(21) Application number: 92110779.3
(22) Date of filing: 26.06.1992
(51) Int. Cl.: C07C 31/20, C07C 29/141, C07C 45/75, C07C 47/19

(54) **Manufacture of neopentyl glycol (IV)**
Herstellung von Neopentylglykol (IV)
Préparation de néopentylglycol (IV)

(30) Priority: 28.06.1991 US 723097; 08.06.1992 US 894961
(43) Date of publication of application: 13.01.1993
(73) Proprietor: Aristech Chemical Corporation, Pittsburgh, PA 15230-0250 (US)
(72) Inventor: Aiken, John E., Monroeville, Pennsylvania 15146 (US)
(74) Representative: Grünecker, Kinkeldey, Stockmair & Schwanhäusser Anwaltssozietät

(56) References cited:
- EP-A- 0 278 106
- EP-A- 0 484 800
- EP-A- 0 536 377
- BE-A- 647 588
- DE-B- 1 014 089
- FR-A- 1 202 524
- FR-A- 2 019 028
- US-A- 4 094 914

## Description

This invention relates to the manufacture of neopentyl glycol (NPG) from isobutyraldehyde and formaldehyde; in particular, it employs formaldehyde in the form of paraformaldehyde or as an aqueous solution, and is restricted to the use of certain specified steps and conditions, notably the presence of a lower alcohol, in the hydrogenation or distillation of the product of the reaction of formaldehyde and isobutyraldehyde (IBAL).

### Defining Terms: Paraformaldehyde, Aqueous Formaldehyde, and Formaldehyde

Commercially, formaldehyde is available in either of two forms, paraformaldehyde or as an aqueous solution (referred to herein as aqueous formaldehyde). Paraformaldehyde is a crystalline solid consisting of a linear polymeric form of formaldehyde of the molecular formula, HO(CH₂)ₙH where n = 8-100. Aqueous formaldehyde consists predominently as formaldehyde in its monomeric form. On standing, it will gradually react with itself forming oligomeric formaldehyde and paraformaldehyde. This is commonly inhibited by adding up to 15% methanol as a stabilizer. The term formaldehyde will be used henceforth to designate that either formaldehyde in the form of paraformaldehyde or aqueous formaldehyde is acceptable (or combinations thereof) unless otherwise specified.

It is known to react paraformaldehyde with isobutyraldehyde to make a product comprising in the case of US-A-5 144 088 (Serial No. 691,927), hydroxypivaldehyde (HPA), and in Serial No. 716,177, 3-hydroxy-2,2-dimethylpropyl hydroxypivalate. The products are hydrogenated to make NPG. The above two documents describe different catalyst systems for the aldol reaction; the hydrogenation step is distinguished primarily by the fact that hydrogenolysis of the indigenous ester impurities is achieved under relatively mild hydrogenation conditions. By this process, the feed material for hydrogenation, i.e. the reaction product of the aldol step, after dissolution in a suitable alcohol, may be fed directly to the hydrogenation step and the resultant NPG product may be recovered by distillation. As described in US-A-5 146 012 (Serial No. 723,097), this was thought to be due in part to the use of paraformaldehyde as the formaldehyde reactant, which greatly reduces the presence of water and avoids other complications, particularly in the generation of waste. The present application is an improvement on the previous inventions, and in the manufacture of NPG generally, in that the present application recognizes that the hydrogenation process described in US-A-5 146 012 (Serial No. 723,097) is admirably suited for input streams with or without the presence of significant amounts of water. References which employ copper chromite and other hydrogenation catalysts with the conventional aqueous formaldehyde system which do not teach the addition of a suitable alcohol solvent prior to hydrogenation may be exemplified by US-A-4,855,515, which recites the historical development of the reaction and emphasizes the use of a particular catalyst in the hydrogenation step. US-A-3,808,280 discloses the use of triethylamine as a catalyst for the aqueous formaldehyde/isobutyraldehyde reaction.

Paraformaldehyde is used by Snam S.p.A. in GB-A-1,017,618 to react with IBAL in the presence of a tertiary amine to produce a reaction product containing apparently predominantly HPA which may be hydrogenated to NPG. However, the instant invention teaches the addition of a suitable alcohol solvent prior to hydrogenation or distillation which produces a high purity NPG product by simple distillation, obviating the need for additional expensive purification steps.

While zur Hausen et al, US-A-4,250,337 may use the aldol reaction product directly in their hydrogenation step, they do not use an alcohol in the hydrogenation step to promote hydrogenolysis. As a result of the use of alcohol, our invention achieves high NPG purities together with high yields unlike the aforementioned patent which can only achieve equivalent purities at uneconomical yields.

Other prior art processes which emphasize the hydrogenation step include US-A-4,094,914 to Rottig et al and US-A-4,933,473 to Ninomiya et al. Ninomiya et al especially recognize the formation of the HPA dimer in the aldol reaction product. Rottig et al use alcohols only in a vapor phase hydrogenation which does not include hydrogenolysis, and do not recognize or demonstrate ester hydrogenolysis.

US-A-4,855,515 claims the use of a manganese promoted copper chromite which allows for efficient hydrogenation at pressures as low as 3.55 MPa (500 psig) in the presence of the amine catalyst and water. However, the product purification is complicated and expensive. The excess IBAL used in the aldol section must be removed before hydrogenation by distillation. After hydrogenation, the effluent must be treated with caustic to saponify the NPG esters and to neutralize amine salts. The sodium salts formed must then be separated from the product before rectification, and an extractive distillation is also necessary to separate recycle IBAL and triethylamine from water, methanol and isobutanol. The purity of the NPG so derived is not specified.

DE-B-1014089 discloses a process for producing neopentyl glycol from isobutyraldehyde and aqueous formaldehyde. No tertiary amine catalyst is used in the aldol reaction.

EP-A-0484800 discloses a process for producing neopentyl glycol which comprises hydrogenating hydroxypivaldehyde in the presence of a catalyst comprising copper, zinc and zirconium whereby the hydroxypivaldehyde is prepared by e.g. the reaction of isobutyraldehyde with formaldehyde in the presence of a tertiary amine and the hydrogenation is conducted in the presence of e.g. an alcohol.

The object of the present invention was to provide a simple and cheap method of making neopentyl glycol having a high purity.

This object has been solved by providing a method of making neopentyl glycol comprising the steps of:
a) reacting formaldehyde with isobutyraldehyde via an aldol reaction in the presence of a tertiary amine catalyst of the formula R¹R²R³N, wherein R¹, R² and R³ are independently selected from alkyl groups having from 1 to 5 carbon atoms and aryl groups and R¹ and R² may form a substituted or unsubstituted cyclic ring having from 5 to 10 carbon atoms,
b) hydrogenating the reaction product obtained in step (a) in the liquid phase at a pressure ranging from 3.45 x 10³ kPa (500 psi) to 20.69 x 10³ kPa (3000 psi) and at a temperature ranging from 100 to 200 °C in the presence of a copper chromite catalyst and in the presence of an alcohol of the formula RR'CHOH, wherein R and R' are independently selected from hydrogen and alkyl groups having from 1 to 5 carbon atoms and have a total of no more than 5 carbon atoms, the amount of said alcohol being at least 20 wt %, based on the mixture of the alcohol and the aldol reaction product, and
c) recovering the neopentyl glycol obtained in step (b),
with the proviso that no metal oxide catalyst is used in the aldol reaction of step (a) and that the hydrogenation catalyst used in step (b) is not a catalyst comprising copper, chromium and manganese or a catalyst containing zinc and zirconium.

The recovered NPG product has a purity of at least 99%. We prefer to use a hydrogenation feed containing 20 to 90% alcohol and 0-40% water, preferably 30-60% alcohol and 0-20% water, and most preferably 50% alcohol and 1-15% water; the preferred alcohol is methanol. In contrast to the concept of US-A-5 146 012 (Serial No. 723,097), we now believe the most important aspect of the process is the presence of alcohol regardless of the presence or absence of water during the hydrogenation step.

Our invention will be described in detail in connection with the examples to follow.

In the discussion below, it is important to keep in mind the basics of the reactions discussed. First is the aldol reaction: 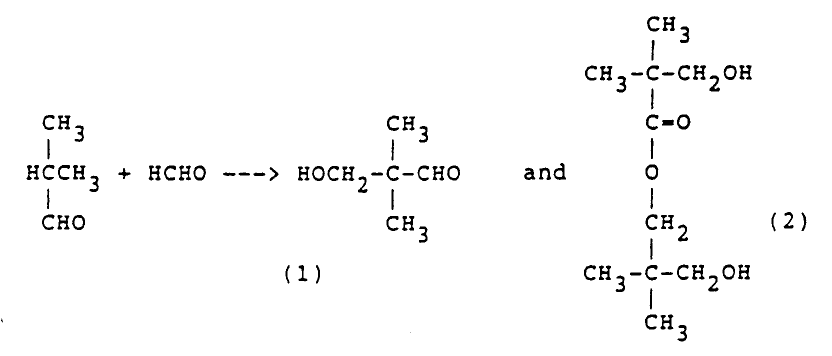 Product (1), which is the major product is HPA. Product (2) corresponds to 3-hydroxy-2,2-dimethylpropyl hydroxypivalate, which will be referred to herein as "HNHP" which stands for hydroxyneopentyl hydroxypivalate. HNHP is generally a minor product made by the Tishchenko reaction of HPA. In the presence of an appropriate catalyst, pressure, heat and hydrogen, the reaction product including both HPA and HNHP is hydrogenated to form NPG:

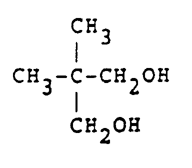

The conventional processes for making NPG by the above route, and which do not add an alcohol in the hydrogenation or distillation step, have the disadvantages of a significant incidence of ester and acid impurities not readily separable from NPG and which must be saponified and neutralized, creating a waste stream, an extraneous extraction step on the waste stream to increase efficiency, and an expensive and complicated purification step. By using the process of our invention, with or without paraformaldehyde, rather than the prior art formaldehyde process, the just-recited disadvantages are obviated. Our process not only avoids these disadvantages but also conducts the hydrogenation step with much greater efficiency. The purity of the NPG product obtained by simple distillation following the teachings of this invention is higher than that obtained by prior art.

Additionally, it has been demonstrated, as will be described herein, that generation of HPA using paraformaldehyde followed by catalytic hydrogenation in an alcoholic solvent can be performed under conditions of hydrogenolysis so that ester impurities are reduced to their corresponding alcohols. NPG product can be obtained from the reduced effluents at >99.5% purity by simple distillation in yields at least comparable to the conventional NPG process using aqueous formaldehyde as described in US-A- 4,855,515.

The high NPG purity of the hydrogenated aldol effluent makes it feasible to eliminate three processing steps: caustic treatment, a distillation or evaporation step such as wiped-film evaporation, and IBAL extraction (see US-A- 4,935,555 to Elias et al). We have produced NPG product in purities greater than 99.5% using this simplified processing scheme. Finally, we have demonstrated to our great surprise that high NPG purities can be achieved at hydrogenation pressures as low as 3.55 MPa (500 psig) H₂ using a conventional copper chromite catalyst.

### "Specific" Procedure using Paraformaldehyde

A specific reaction using paraformaldehyde may be described as follows: The reaction is performed in a reflux apparatus wherein 1.00 equivalent of IBAL, 1.06 equivalents of paraformaldehyde, and 0.04 to 0.05 equivalents of triethylamine have been placed under an inert atmosphere. The reaction mixture is stirred at 60-80°C for 5-6 hours or until the reaction is completed. The clear liquid is diluted in a solvent such as methanol and hydrogenated by passing the reaction solution over a conventional copper chromite catalyst at about 160°C and about 6.89 MPa (1000 psi). High purity NPG product (>99.5%) is recovered in high yield by distillation.

### "Specific" Procedure using Aqueous Formaldehyde

Another specific reaction using aqueous formaldehyde may be described as follows: The reaction is performed in a reflux apparatus wherein 1.00 equivalent of IBAL, 1.00 equivalents of aqueous formaldehyde, and 0.04 to 0.08 equivalents of triethylamine have been placed under an inert atmosphere. The reaction mixture is stirred at 60-80°C for 2-4 hours or until the reaction is completed. The clear liquid is diluted in a solvent such as methanol and hydrogenated by passing the reaction solution over a conventional copper chromite catalyst at about 160°C and about 6.89 MPa (1000 psi). High purity NPG product (>99.5%) is recovered in high yield by distillation.

### "General" Procedure using Formaldehyde

More generally, with 1 equivalent of IBAL we may place in a reaction vessel from 0.5 to 2 equivalents of formaldehyde and 0.001 to 0.1 equivalents (preferably 0.005 to 0.1 equivalents) of a tertiary amine catalyst. The reaction mixture is stirred at 60-80°C until most of the IBAL is consumed. The resulting solution is diluted in a solvent such as methanol and hydrogenated using a conventional copper chromite hydrogenation catalyst. NPG product is obtained by distillation.

We use as catalysts any tertiary amines of the general formula R¹R²R³N where R¹, R², and R³ are independently selected from alkyl groups having from one to five carbon atoms and aryl groups and R¹ and R² may form a substituted or unsubstituted cyclic ring having from 5 to 10 carbon atoms. As is known in the art, if the amine chosen has a boiling point lower than the boiling point (reflux temperature) of IBAL, pressure may be necessary.

Following are several examples of the invention:

### Example 1

Hydroxyneopentyl hydroxypivalate (HNHP) hydrogenolysis compared to methyl isobutyrate hydrogenolysis:

The following solutions were prepared:

| | | |
|---|---|---|
| (A) | NPG | 47.6 wt.% |
| | HNHP | 2.4 wt.% |
| (B) | triethylamine | 2.3 wt.% |
| | methanol | 47.6 wt.% |
| | methyl isobutyrate | 5 wt.% |
| | methanol | 95 wt.% |

A batch hydrogenation was performed on each solution using 1.4 wt.% stabilized copper chromite at 150°C for 1 hour at 7.0 MPa (1000 psig) H₂. Ester hydrogenolysis was monitored. The results follow in Table I. These results are surprising in that the ester impurities indigenous to the process in this invention are more easily hydrogenolyzed than a typical ester such as methyl isobutyrate; they are also surprising in that we are able to hydrogenate easily at relatively low pressures and temperatures. This allows the recovery of high purity NPG product by distillation.

**Table I**

| Ester | % Hydrogenolysis |
|---|---|
| HNHP | 65.7 % |
| Methyl isobutyrate | 1.4 % |

### Example 2

Isobutyraldehyde (2000.0 g, 27.74 mol), paraformaldehyde (929.3 g, 29.40 mol), and triethylamine (140.3 g, 1.39 mol) were charged with stirring into a 5 ℓ roundbottom flask fitted with a reflux condenser. The apparatus was lowered into a water bath (50°C). The bath was heated to a temperature of 80°C over a period of 1.5hours. The reaction was terminated (6hours) and the clear, faintly yellowish aldol effluent was diluted in methanol to make a 50 wt.% aldol in methanol solution. The hydrogenation was performed by passing the methanolic aldol effluent upward through a fixed-bed of stabilized copper chromite at 160°C, 0.5 LHSV, and 7.0 MPa (1000 psig) H₂ at >5:1 mole ratio H₂:HPA. The results are as shown:

| Components | Hydrogenated Solution (Wt %) | *Neopentyl Glycol Purity |
|---|---|---|
| CH₃OH | 48.79 | |
| IBA | 1.00 | |
| IBacid | 0.00 | |
| TEA | 2.13 | |
| HPA | 0.00 | |
| NPG | 44.93 | 99.67% |
| esters | 0.15 | |
| HNHP | 0.27 | |
| Water | 2.72 | |

| | | |
|---|---|---|
| *NPG purities calculated by GC on a "lights-free" (CH₃OH, IBA, TEA), "HNHP-free" basis. IBA = isobutyl alcohol IBacid = isobutyric acid TEA = triethylamine HPA = hydroxypivaldehyde NPG = neopentyl glycol esters = e.g., neopentyl glycol monoformate, neopentyl glycol monoisobutyrate HNHP = hydroxyneopentyl hydroxypivalate | | |

In order to demonstrate the effect of various concentrations of alcohol and water during hydrogenolysis, the following experiments were performed, with the results shown in Tables II and III:

### Example 3

Isobutyraldehyde (1383.0 g, 19.18 mol), paraformaldehyde (670.9 g, 20.33 mol), methanol (349.1 g), and triethylamine (97 g, .96 mol) were charged with stirring into a 5ℓ roundbottom flask fitted with a reflux condenser and overhead stirrer. The methanol was added to simulate the methanol contained in the triethylamine recycle stream in a continuous process. The apparatus was held in a water bath (50°C). After addition of the reagents, the bath was heated to a temperature of 80°C over a period of 1.5 hours. Over 95% of the IBAL was reacted when the reaction was terminated after 5-1/2 hours and the clear, faintly yellowish aldol effluent was diluted in methanol to make a 50 wt % aldol in methanol solution. The hydrogenation was performed by passing the methanolic aldol effluent upward through a fixed-bed of stabilized copper chromite at 160°C, 0.5 LHSV, and 7.0 MPa (1000 psig) at a 16:1 mole ratio H₂:HPA. The resultant hydrogenation product was batch distilled in a 30-tray Oldershaw column at a 2:1 reflux ratio to recover a high purity neopentyl glycol product. The results are as shown:

| Components | Hydrogenated Solution (Wt %) | Distilled Product (Wt %) |
|---|---|---|
| Methanol | 49.65 | |
| Isobutanol | 1.47 | |
| TEA | 2.15 | |
| NPG | 43.63 | 99.63 |
| esters | 0.26 | 0.37 |
| HNHP | 0.67 | |
| Water | 2.05 | |
| TEA = triethylamine NPG = neopentyl glycol esters = e.g., neopentyl glycol monoformate, neopentyl glycol monoisobutyrate HNHP = hydroxyneopentyl hydroxypivalate | | |

### Example 4

(A) As a control, 1229 g (16.5 mol) of isobutyraldehyde, 1315.0 g (16.2 mol) of 37% aqueous formaldehyde, 96.3 g (.95 mol) of triethylamine, and 236.7 g of methanol were charged with stirring into a 5ℓ flask equipped with an overhead stirrer and a reflux condenser. The methanol was added for two purposes: maintain the aldol reaction as a single liquid phase and simulate the methanol present due to recycle of the triethylamine stream in a continuous process. The apparatus was held in a water bath at 50°C. After addition of the reagents, the bath was heated to 60°C. Over 95% of the IBAL was converted when the reaction was terminated after 4 hours. The hydrogenation was performed by passing the undiluted aldol effluent upward through a fixed-bed of stabilized copper chromite at 160°C, 1.3 LHSV, and 7.0 MPa (1000 psig). The resultant hydrogenation product was batch distilled in a 30-tray Oldershaw column at a 2:1 reflux ratio to recover the neopentyl glycol product.
(B) The aldol reaction and hydrogenation described in (A) was repeated except that the hydrogenation effluent was diluted with 2332.4 g of methanol prior to distillation to make a 50 wt % methanol solution. The resultant hydrogenation product was batch distilled in a 30-tray Oldershaw column at a 2:1 reflux ratio to recover the neopentyl glycol product.

The results from the two experiments are summarized in Table II. The data demonstrate the advantage of adding a large amount of an alcoholic solvent after hydrogenation, namely a decrease in neopentyl glycol ester impurities made during the distillation. Without the addition of the alcohol, a much larger amount of neopentyl glycol is converted to impurities.

**Table II**

| Example % Methanol | 4(A) 9.5% | 4(B) 50% (after hydrogenation) |
|---|---|---|
| Wt % water in hydrogenation | 26.4% | 26.4% |
| Crude NPG Purity* | 98.86% | 98.86% |
| Reaction Selectivity** | 83.26% | 84.66% |
| Total Impurities made*** | 96.01 | 57.88 |
| Product recovery**** | 44% | 65% |

| | | |
|---|---|---|
| * Crude NPG purity is the purity of the neopentyl glycol product estimated on a "lights-free" (isobutyl alcohol, triethylamine, and methanol) and hydroxyneopentyl hydroxypivalate-free weight % basis. | | |
| ** Reaction selectivity is the weight percent of neopentyl glycol based on the total IBAL plus formaldehyde reaction products contained in the hydrogenation effluent. | | |
| *** Total impurities made is the grams per 1000 grams neopentyl glycol of neopentyl glycol esters and HPA side reaction products made during the aldol and hydrogenation reactions and distillation. | | |
| **** Product recovery is the percent of neopentyl glycol recovered as 99.5% pure product in the distillation. | | |

### Example 5

(A) The aldol reaction described in Example 4(A) was repeated except that the aldol effluent was diluted with 2332.4 g of methanol prior to hydrogenation rather than following hydrogenation to make a 50 wt % aldol in methanol solution. The hydrogenation was performed by passing the methanolic aldol effluent upward through a fixed-bed of stabilized copper chromite at 160°C, 1.3 LHSV, and 7.0 MPa (1000 psig). The resultant hydrogenation product was batch distilled in a 30-tray Oldershaw column at a 2:1 reflux ratio to recover high purity neopentyl glycol product.
(B) The aldol reaction described in (A) was repeated except that 2332.4 g of isopropanol was used to dilute the aldol reaction effluent to make a 50 wt % aldol in alcohol solution. The diluted effluent was then hydrogenated at 160°C, 7.0 MPa (1000 psig), and 1.3 LHSV. The hydrogenation effluent was then batch distilled in a 30-tray Oldershaw column at a 2:1 reflux ratio to recover high purity neopentyl glycol product.
(C) The aldol reaction described in (A) was repeated except that 2332.4 g of n-butanol was used to dilute the aldol reaction effluent to make a 50 wt % aldol in alcohol solution. The diluted effluent was then hydrogenated at 160°C, 7.0 MPa (1000 psig), and 1.3 LHSV. The hydrogenation effluent was then batch distilled in a 30-tray Oldershaw column at a 2:1 reflux ratio to recover high purity neopentyl glycol product.

The data for the three experiments plus the control from Example 4 are summarized in Table III. The results demonstrate the surprising improvement in neopentyl glycol purity and yields by the addition of a large amount of a suitable alcohol solvent prior to hydrogenation. By following the procedure taught by this invention, the alcohol solvent is used to enhance hydrogenolysis of neopentyl glycol esters as well as undergoing ester interchange to form lower-boiling, easily separable esters. Thus, high purity neopentyl glycol product can be obtained without the need for expensive purification steps such as a caustic treatment. The hydrogenation step described in this improved process would be applicable to an aldol effluent made using either aqueous formaldehyde or paraformaldehyde.

By a suitable alcohol solvent, we mean an alcohol of the formula RR'CHOH wherein R and R' are independently selected from hydrogen and alkyl groups having from one to five carbon atoms. We prefer that the hydrogenolysis feed contains 20 to 90 wt % alcohol, preferably 30 to 60 wt % alcohol, and most preferably 50 wt % alcohol. The alcohol can be recycled prior to recovering the neopentyl glycol.

**Table III**

| Example Solvent | 4(A) Control 9.5 wt% Methanol | 5(A) 50 wt% Methanol | 5(B) 50 wt% 2-Propanol | 5(C) 50 wt% n-Butanol |
|---|---|---|---|---|
| Wt% water in hydrogenation | 26.4% | 14.8% | 14.8% | 14.8% |
| Crude NPG Purity* | 98.86% | 99.67% | 99.70% | 99.29% |
| Distilled NPG Purity | 99.51% | 99.85% | 99.75% | 99.70% |
| Reaction Selectivity** | 83.26% | 93.04% | 91.41% | 89.91% |
| Total Impurities made*** | 96.01 | 20.57 | 27.07 | 49.52 |

| | | | | |
|---|---|---|---|---|
| * Crude NPG purity is the purity of the neopentyl glycol product estimated on a "lights-free" (isobutyl alcohol, triethylamine, and methanol) and hydroxyneopentyl hydroxypivalate-free weight % basis. | | | | |
| ** Reaction selectivity is the percent of neopentyl glycol based on the total IBAL plus formaldehyde reaction products contained in the hydrogenation effluent. | | | | |
| *** Total impurities made is the grams per 1000 grams neopentyl glycol of neopentyl glycol esters and HPA side reaction products made during the aldol and hydrogenation reactions and distillation. | | | | |

### Example 6

122.9 g of IBAL, 131.5 g of 37% aqueous formaldehyde, 9.6 g of triethylamine, and 23.7 g of methanol were charged with stirring into a flask equipped with a reflux condenser. The apparatus was held in a heated bath at 50°C. After addition of the reagents, the bath was heated to 60°C. The reaction was terminated after 4 hours and the aldol product was diluted with 2317.0 g of methanol to yield a 90 wt % methanol/10 wt % aldol solution. The hydrogenation was performed by passing the methanolic solution upward through a fixed-bed of stabilized copper chromite at 160°C, 1.6 LHSV, and 7.0 MPa (1000 psig) H₂. The resultant material was batch distilled to recover the NPG product and the results are as shown:

| Components | Hydrogenated Solution (Wt %) | Neopentyl Glycol Product (Wt %) |
|---|---|---|
| Methanol | 90.38 | |
| Isobutanol | 0.24 | |
| TEA | 0.33 | |
| NPG | 5.78 | 99.60 |
| esters | 0.03 | 0.40 |
| HNHP | 0.05 | |
| Water | 3.17 | |
| TEA = triethylamine NPG = neopentyl glycol esters = e.g., neopentyl glycol monoformate, neopentyl glycol monoisobutyrate HNHP = hydroxyneopentyl hydroxypivalate | | |

### Example 7

Isobutyraldehyde (1106.4 g), paraformaldehyde (536.7 g mol), methanol (279.3 g), and triethylamine (77.6 g) were charged with stirring into a 5ℓ roundbottom flask fitted with a reflux condenser. The methanol was added to simulate the methanol contained in the triethylamine recycle stream in a continuous process. The apparatus was held in a water bath at 50°C. After addition of the reagents, the bath was heated to a temperature of 80°C over a period of 1.5 hours. The reaction was terminated after 5-1/2 hours and the aldol effluent was diluted in methanol to make a 50 wt % aldol in methanol solution. An additional 2000 g of water was added to the methanolic solution to increase the total water content to 37.6 wt % (based on the entire feed to the hydrogenation step). The hydrogenation was performed by passing the methanolic aldol effluent upward through a fixed-bed of stabilized copper chromite at 160°C, 1.6 LHSV, and 7.0 MPa (1000 psig). The resultant hydrogenation product was batch distilled in a 30-tray Oldershaw column at a 2:1 reflux ratio to recover a high purity neopentyl glycol product. The results are as shown:

| Components | Hydrogenated Solution (Wt %) | Neopentyl Glycol Product (Wt %) |
|---|---|---|
| Methanol | 32.36 | |
| Isobutanol | 2.03 | |
| TEA | 1.04 | |
| NPG | 25.31 | 99.53 |
| esters | 0.48 | 0.47 |
| HNHP | 0.70 | |
| Water | 37.65 | |
| TEA = triethylamine NPG = neopentyl glycol esters = e.g., neopentyl glycol monoformate, neopentyl glycol monoisobutyrate HNHP = hydroxyneopentyl hydroxypivalate | | |

### Example 8

1229 g (16.5 mol) of isobutyraldehyde, 1315.0 g (16.2 mol) of 37% aqueous formaldehyde, 96.3 g (.95 mol) of triethylamine, and 236.7 g of methanol were charged with stirring into a 5ℓ flask equipped with an overhead stirrer and a reflux condenser. The apparatus was held in a water bath at 50°C. After addition of the reagents, the bath was heated to 60°C. The reaction was terminated after 4 hours after which the aldol effluent was diluted with 2332.4 g of methanol to make a 50 wt % methanol solution. The hydrogenation was performed by passing the methanolic aldol effluent upward through a fixed-bed of stabilized copper chromite at 160°C, 1.3 LHSV, and 3.55 MPa (500 psig). The results are as shown:

| Components | Hydrogenated Solution (Wt %) | Crude Neopentyl Glycol Purity* (GC area %) |
|---|---|---|
| CH₃OH | 44.58 | |
| IBA | 2.48 | |
| IBacid | 0.00 | |
| TEA | 1.43 | |
| HPA | 0.00 | |
| NPG | 24.97 | 99.41 |
| esters | 0.33 | 0.59 |
| HNHP | 0.62 | |
| Water | 25.60 | |

| | | |
|---|---|---|
| * Neopentyl glycol purity is calculated by gas chromatograph area % on a "lights-free" (methanol, isobutanol, TEA) and "HNHP-free" basis prior to distillation. IBA = isobutyl alcohol IBacid = isobutyric acid TEA = triethylamine NPG = neopentyl glycol esters = e.g., neopentyl glycol monoformate, neopentyl glycol monoisobutyrate HNHP = hydroxyneopentyl hydroxypivalate | | |

## Claims

1. A method of making neopentyl glycol comprising the steps of:
a) reacting formaldehyde with isobutyraldehyde via an aldol reaction in the presence of a tertiary amine catalyst of the formula R¹R²R³N, wherein R¹, R² and R³ are independently selected from alkyl groups having from 1 to 5 carbon atoms and aryl groups and R¹ and R² may form a substituted or unsubstituted cyclic ring having from 5 to 10 carbon atoms;
b) hydrogenating the reaction product obtained in step (a) in the liquid phase at a pressure ranging from 3.45 x 10³ kPa (500 psi) to 20.69 x 10³ kPa (3000 psi) and at a temperature ranging from 100 to 200°C in the presence of a copper chromite catalyst and in the presence of an alcohol of the formula RR'CHOH, wherein R and R' are independently selected from hydrogen and alkyl groups having from 1 to 5 carbon atoms and have a total of no more than 5 carbon atoms, the amount of said alcohol being at least 20 wt.%, based on the mixture of the alcohol and the aldol reaction product; and
c) recovering the neopentyl glycol obtained in step (b),
with the proviso that no metal oxide catalyst is used in the aldol reaction of step (a) and that the hydrogenation catalyst used in step (b) is not a catalyst comprising copper, chromium and manganese or a catalyst containing zinc and zirconium.

2. The method of claim 1, wherein the formaldehyde is in the form of paraformaldehyde.

3. The method of claim 1, wherein the formaldehyde is aqueous formaldehyde.

4. The method of claim 1, wherein the alcohol comprises methanol.

5. The method of claim 1, wherein the reaction of isobutyraldehyde with formaldehyde employs a molar ratio of formaldehyde relative to isobutyraldehyde of 0.5:1 to 2:1.

6. The method of claim 1, wherein the alcohol content of the aldol-reaction product mixture is no greater than 90 % by weight and water is additionally present in the hydrogenation step in an amount no greater than 40 % by weight based on the total of the reaction product, alcohol and water.

7. The method of claim 1, wherein the alcohol is recovered prior to recovering the neopentyl glycol and is recycled.

8. The method of claim 1, wherein the neopentyl glycol is recovered by distillation.

9. The method of claim 6, wherein the alcohol is present during the hydrogenation reaction in an amount from 30 to 60 % by weight of the mixture with the aldol reaction product and the water is present in an amount from 0 to 20 % by weight.

10. The method of claim 9, wherein the alcohol is present during the hydrogenation reaction in an amount of 50 % by weight of the mixture with the aldol reaction product and the water is present in an amount from 1 to 15 % by weight.

## Patentansprüche

1. Verfahren zur Herstellung von Neopentylglycol, umfassend die folgenden Verfahrensschritte:
a) Umsetzen von Formaldehyd mit Isobutylaldehyd über eine Aldolreaktion in Gegenwart eines Katalysators aus einem tertiären Amid der Formel R¹R²R³N, worin R¹, R² und R³ unabhängig voneinander ausgewählt sind aus Alkylgruppen mit 1 bis 5 Kohlenstoffatomen und Arylgruppen, wobei R¹ und R² einen substituierten oder unsubstituierten cyclischen Ring mit 5 bis 10 Kohlenstoffatomen bilden können;
b) Hydrieren des in Schritt (a) erhaltenen Reaktionsproduktes in der Flüssigphase bei einem Druck im Bereich von 3,45 x 10³ kPa (500 psi) bis 20,69 x 10³ kPa (3000 psi) und bei einer Temperatur im Bereich von 100 bis 200°C in Gegenwart eines Kupferchromit-Katalysators und in Gegenwart eines Alkohols der Formel RR'CHOH, wobei R und R' unabhängig voneinander ausgewählt sind aus Wasser-. stoff und Alkylgruppen mit 1 bis 5 Kohlenstoffatomen, wobei R und R' zusammen nicht mehr als 5 Kohlenstoffatome besitzen und wobei die Mange des Alkohols mindestens 20 Gew.-% beträgt, bezogen auf das Gemisch aus dem Alkohol und dem Aldolreaktionsprodukt, und
c) Gewinnen des in Schritt (b) erhaltenen Neopentylglycols,
mit der Maßgabe, daß in der Aldolreaktion von Schritt (a) kein Metalloxidkatalysator verwendet wird, und mit der Maßgabe, daß der in Schritt (b) verwendete Hydrierungskatalysator kein Katalysator ist, der Kupfer, Chrom und Mangan enthält, und kein Katalysator ist, der Zink und Zirkon enthält.

2. Verfahren nach Anspruch 1, worin der Formaldehyd in Form von Paraformaldehyd vorliegt.

3. Verfahren nach Anspruch 1, worin der Formaldehyd in Form einer wäßrigen Formaldehydlösung vorliegt.

4. Verfahren nach Anspruch 1, worin der Alkohol Methanol umfaßt.

5. Verfahren nach Anspruch 1, worin bei der Umsetzung von Isobutylaldehyd mit Formaldehyd ein molares Verhältnis von Formaldehyd zu Isobutylaldehyd von 0,5:1 bis 2:1 verwendet wird.

6. Verfahren nach Anspruch 1, worin der Gehalt an Alkohol in den Gemisch mit dem Aldolreaktionsprodukt nicht mehr als 90 Gew.-% beträgt, und beim Hydrieren zusätzlich Wasser in einer Menge von nicht mehr als 40 Gew.-% vorliegt, bezogen auf die Gesamtmenge des Reaktionsproduktes_{,} des Alkohols und des Wassers.

7. Verfahren nach Anspruch 1, worin der Alkohol zurückgewonnen wird, bevor das Neopentylglycol gewonnen wird, und zurückgeführt wird.

8. Verfahren nach Anspruch 1, worin das Neopentylglycol durch Destillation gewonnen wird.

9. Verfahren nach Anspruch 6, worin der Alkohol während der Hydrierung in einer Menge im Bereich von 30 bis 60 Gew.-% vorliegt, bezogen auf das Gemisch mit den Aldolreaktionsprodukt, und das Wasser in einer Menge im Bereich von 0 bis 20 Gew.-% vorliegt.

10. Verfahren nach Anspruch 9 worin der Alkohol während der Hydrierung in einer Menge von 50 Gew.-% vorliegt, bezogen auf das Gemisch mit dem Aldolreaktionsprodukt, und das Wasser in einer Menge im Bereich von 1 bis 15 Gew.-% vorliegt.

## Revendications

1. Procédé de fabrication de néopentylglycol comprenant les étapes consistant
a) à faire réagir du formaldéhyde avec de l'isobutyraldéhyde par l'intermédiaire d'une réaction de formation d'aldol en présence d'un catalyseur d'amine tertiaire de la formule R¹R²R³N, dans laquelle R¹, R² et R³ sont indépendamment choisis parmi des groupes alkyles ayant de 1 à 5 atomes de carbone et des groupes aryles et R¹ et R² peuvent former un noyau cyclique substitué ou non substitué ayant de 5 à 10 atomes de carbone,
b) à hydrogéner le produit de réaction obtenu dans l'étape (a) dans la phase liquide à une pression comprise entre 3,45 x 10³ kPa (500 psi) et 20,69 x 10³ kPa (3000 psi) et à une température comprise entre 100 et 200°C en présence d'un catalyseur de chromite de cuivre et en présence d'un alcool de la formule RR'CHOH, dans laquelle R et R' sont indépendamment choisis parmi un atome d'hydrogène et des groupes alkyles ayant de 1 à 5 atomes de carbone et présentent un total qui n'est pas supérieur à 5 atomes de carbone, la quantité dudit alcool étant d'au moins 20 % en poids, rapportés au mélange de l'alcool et du produit de réaction de formation d'aldol, et
c) à récupérer le néopentylglycol obtenu dans l'étape (b),
à la condition que l'on n'utilise aucun catalyseur d'oxyde métallique dans la réaction de formation d'aldol de l'étape (a) et que le catalyseur d'hydrogénation utilisé dans l'étape (b) n'est pas un catalyseur comprenant du cuivre, du chrome et du manganèse ou un catalyseur contenant du zinc et du zirconium.

2. Procédé selon la revendication 1, dans lequel le formaldéhyde est dans la forme de paraformaldéhyde.

3. Procédé selon la revendication 1, dans lequel le formaldéhyde est du formaldéhyde aqueux.

4. Procédé selon la revendication 1, dans lequel l'alcool comprend du méthanol.

5. Procédé selon la revendication 1, dans lequel la réaction d'isobutyraldéhyde avec du formaldéhyde utilise un rapport molaire du formaldéhyde par rapport à l'isobutyraldéhyde de 0,5:1 à 2:1.

6. Procédé selon la revendication 1, dans lequel la teneur en alcool du mélange du produit de réaction de formation d'aldol n'est pas supérieure à 90 % en poids et de l'eau est de plus présente dans l'étape d'hydrogénation dans une quantité qui n'est pas supérieure à 40 % en poids, rapportés au total du produit de réaction, de l'alcool et de l'eau.

7. Procédé selon la revendication 1, dans lequel l'alcool est récupéré avant la récupération du néopentylglycol et est recyclé.

8. Procédé selon la revendication 1, dans lequel le néopentylglycol est récupéré par distillation.

9. Procédé selon la revendication 6, dans lequel l'alcool est présent pendant la réaction d'hydrogénation dans une quantité de 30 à 60 % en poids du mélange avec le produit de réaction de formation d'aldol et l'eau est présente dans une quantité de 0 à 20 % en poids.

10. Procédé selon la revendication 9, dans lequel l'alcool est présent pendant la réaction d'hydrogénation dans une quantité de 50 % en poids du mélange avec le produit de réaction de formation d'aldol et l'eau est présente dans une quantité de 1 à 15 % en poids.
